# EUROPÄISCHE PATENTANMELDUNG

(11) **EP 3 299 799 A1**
(43) Veröffentlichungstag der Anmeldung: **28.03.2018**
(21) Anmeldenummer: 16190899.1
(22) Anmeldetag: 27.09.2016
(51) Int. Cl.: G01N 21/25, G01N 21/45, G01N 21/77, G01N 33/543

(54) **VERFAHREN UND MESSSYSTEM ZUR MESSUNG MOLEKULARER INTERAKTIONEN AN EINER DÜNNEN SCHICHT**

(71) Anmelder: Berthold Technologies GmbH & Co. KG, 75323 Bad Wildbad (DE)
(72) Erfinder: Schleifenbaum, Frank, 88690 Uhldingen-Mühlhofen (DE)
(74) Vertreter: Patentanwälte Ruff, Wilhelm, Beier, Dauster & Partner mbB

(57) **Zusammenfassung**

Ein Verfahren zur markierungsfreien Messung molekularer Interaktionen an einer dünnen Schicht (DS) umfasst die Schritte: Beleuchten der dünnen Schicht (DS) mit Licht einer Lichtquelle (LQ); Erfassen eines an Grenzflächen der dünnen Schicht reflektierten oder transmittierten Lichtanteils mittels eines Detektors (DET), welcher den erfassten Lichtanteil in elektrische Signale wandelt; und Auswerten der elektrischen Signale zur Ermittlung von Parametern, die die zu untersuchenden Interaktionen charakterisieren. Das Verfahren ist dadurch gekennzeichnet, dass die dünne Schicht (DS) mit polychromatischem Licht bestrahlt wird, das gleichzeitig Lichtanteile aus mehreren separaten Spektralbereichen enthält; ein farbsensitiver ortsauflösender Detektor (DET) verwendet wird, der die an der dünnen Schicht (DS) reflektierten oder durch die dünne Schicht transmittierten Lichtanteile in mehreren mit den Spektralbereichen mindestens teilweise überlappenden Detektionswellenlängenbereichen (D1, D2, D3) ortsaufgelöst detektiert und in separate, von den Intensitäten in den Detektionswellenlängenbereichen abhängige elektrische Signale wandelt; und die Auswertung der Signale eine Analyse von Intensitätsverhältnissen in den Detektionswellenlängenbereichen umfasst.

## Beschreibung

Die Erfindung betrifft ein Verfahren zur markierungsfreien Messung molekularer Interaktionen an einer dünnen Schicht sowie ein zur Durchführung des Verfahrens geeignetes Messsystem.

Interaktionen biologisch aktiver Moleküle nehmen eine zentrale Stellung in biochemischen Prozessen und in der pharmakologischen Wirkstoffforschung ein. Beispielhaft seien Genexpression, Signalübertragung durch Hormone oder Neurotransmitter, Immunabwehr durch Antigen-Antikörper-Bindung oder Enzymreaktionen genannt. Jedem dieser Vorgänge geht eine spezifische Bindung eines Liganden an einen biologischen Rezeptor voraus. Der Nachweis und die Charakterisierung solcher Bindungsereignisse sind für die Aufklärung und das Verständnis von Mechanismus und Funktion biochemischer Prozesse von entscheidender Bedeutung und bilden eine wesentliche Grundlage für die Entwicklung neuartiger pharmakologischer Wirkstoffe.

Manche Verfahren zur Charakterisierung biomolekularer Reaktionen nutzen eine radioaktive Markierung oder einer Fluoreszenzmarkierung eines der beiden oder beider Bindungspartner. Inzwischen wurden auch verschiedene Verfahren zur markierungsfreien Messung biomolekularer Interaktionen entwickelt, die eine direkte Beobachtung von Bindungsereignissen an Oberflächen und damit den Zugang zur thermodynamischen und kinetischen Charakterisierung der beobachteten Vorgänge erlauben.

Zur markierungsfreien Detektion biomolekularer Wechselwirkungen sind unter anderem Verfahren basierend auf der Reflektometrischen Interferenzspektroskopie (RifS) geeignet. Diese optische Detektionsmethode macht sich die Mehrfachreflexion von Licht an dünnen, transparenten Schichten zunutze. Einfallendes Licht wird teilweise an den Grenzflächen (Phasengrenzen) der Schichten aufgrund unterschiedlicher Brechungsindizes reflektiert. Durch die Überlagerung der reflektierten oder transmittierten Lichtanteile werden charakteristische Interferenzerscheinungen erzeugt. Die Änderung der optischen Schichtdicke der untersuchten dünnen Schicht, die z.B. mit der Anlagerung oder der Bindung von Molekülen verbunden sein kann, verursacht eine spektrale Verschiebung des Interferogramms. Diese Verschiebung kann zeitaufgelöst gemessen werden. Hierdurch lassen sich der Anzahl der Bindungen pro Zeiteinheit proportionale Signale ermitteln und daraus kinetische Parameter wie z.B. die Assoziations- oder Dissoziationskonstante oder die Affinitätskonstante bestimmen. Werden diese Messungen bei unterschiedlichen Temperaturen durchgeführt, sind thermodynamische Parameter wie die Änderung der Enthalpie oder der Entropie zugänglich.

Die DE 42 00 088 A1 offenbart ein Beispiel eines derartigen Verfahrens, bei dem polychromatisches Licht einer Xenonhochrucklampe auf eine dünne Schicht eingestrahlt wird und mittels eines Detektors ein Reflexionsspektrum spektral erfasst und die spektrale Verschiebung des Reflexionsspektrums detektiert wird.

Die DE 198 30 727 A1 offenbart eine Weiterentwicklung des in der DE 42 00 088 A1 beschriebenen Verfahrens. Dabei wird quasi-monochromatisches Licht (also Licht aus einem engen Wellenlängenbereich) aus mindestens drei unterschiedlichen engen Wellenlängenbereichen nacheinander auf die dünne Schicht eingestrahlt. Aus den reflektierten Intensitäten an entsprechenden spektralen Stützpunkten kann z.B. unter Verwendung einer Hüllkurve ein Reflexionsspektrum abgeleitet werden.

Die EP 1 805 502 A1 offenbart ein Verfahren der obigen Art, bei dem die auf Reflexionsmessungen basierende Detektion eines aus mehreren Wellenlängen bestehenden Spektrums auf die Detektion nur einer einzigen (optimalen) Wellenlänge reduziert wird und die Veränderungen der dünnen Schicht über die Detektion von Intensitätsänderungen bei dieser Wellenlänge ermittelt werden. Beschrieben wird auch die Ermittlung der für die Auswertung der Messergebnisse optimalen Wellenlänge. Diese entspricht einer Wellenlänge, bei der die Änderung der Lichtintensität nach einer erfolgten Reaktion oder Wechselwirkung an oder in der dünnen Schicht am größten ist. Als Vorteile werden unter anderem angegeben, dass das Verfahren eine Parallelisierung der Messung und eine Miniaturisierung des Messaufbaus ermöglicht. Außerdem können die Messergebnisse wesentlich schneller und einfacher als bei bekannten Verfahren, praktisch on-line, ausgewertet werden.

Das Patent EP 1 685 367 B1 offenbart eine Anordnung zur Verwendung beim Nachweis eines Analyten in einer Probe auf Basis von Interferenz. Die Anordnung umfasst eine optische Faser mit einer Faserspitze und ein optisches Element, das an die Faserspitze gebunden ist, wobei das optische Element in der Größe der Faserspitze entspricht und zum Koppeln mit einer Lichtquelle über die Faser angepasst ist. Das optische Element umfasst ein transparentes Material, eine erste Reflexionsoberfläche und eine zweite Reflexionsoberfläche, die durch das transparente Material um einen Abstand von mindestens 50 nm von der ersten Reflexionsoberfläche getrennt ist. Die erste Reflexionsoberfläche weist eine Schicht aus Analyt-Bindungsmolekülen auf. Eine Interferenz zwischen Licht, das in die Faser von den ersten und zweiten Reflexionsoberflächen reflektiert wird, variiert, wenn der Analyt in der Probe an die Analyt-Bindungsmoleküle bindet. Die zweite Reflexionsoberfläche umfasst eine Schicht aus Material mit einem Brechungsindex, der größer ist als der Brechungsindex des transparenten Materials. Mit einem geeigneten Detektor können Änderungen der spektralen Intensitätsverhältnisse an der Faserspitze simultan in mindestens zwei unterschiedlichen Spektralbereichen erfasst werden. Eine Bindung wird nachgewiesen über die Bestimmung des Phasenversatzes des Interferenzspektrums.

### AUFGABE UND LÖSUNG

Die Aufgabe der vorliegenden Erfindung besteht darin, ein robustes Verfahren der eingangs erwähnten Art zur Verfügung zu stellen, das es ermöglicht, molekulare Bindungen hoch-parallelisiert und markierungsfrei mittels eines interferometrischen Verfahrens mit hoher Empfindlichkeit zu detektieren. Es ist eine weitere Aufgabe, ein zur Durchführung des Verfahrens geeignetes Messsystem bereitzustellen.

Diese Aufgabe wird gelöst durch ein Verfahren mit den Merkmalen von Anspruch 1 sowie durch ein Messsystem mit den Merkmalen von Anspruch 12. Vorteilhafte Weiterbildungen sind in den abhängigen Ansprüchen angegeben. Der Wortlaut sämtlicher Ansprüche wird durch Bezugnahme zum Inhalt der Beschreibung gemacht.

Bei der Durchführung des Verfahrens wird die dünne Schicht mit polychromatischem Licht bestrahlt. Polychromatisches Licht ist elektromagnetische Strahlung, die gleichzeitig bzw. simultan Lichtanteile aus mehreren separaten Spektralbereichen enthält. Die Spektralbereiche sind insoweit separat, als sie einander nicht überlappen. Die Spektralbereiche können unmittelbar aneinander anschließen, so dass ein kontinuierliches Spektrum vorliegt. Es ist auch möglich, dass die Spektralbereiche mit gegenseitigem Abstand zueinander liegen, so dass ein diskontinuierliches Spektrum (mit Lücken zwischen den genutzten Spektralbereichen) vorliegt. In Sinne dieser Anmeldung bezeichnet der Begriff "Licht" elektromagnetische Strahlung aus dem sichtbaren Wellenlängenbereich (VIS) sowie angrenzenden Wellenlängenbereichen im Ultraviolettbereich (UV) und im nahen Infrarotbereich (NIR).

Zur Detektion des an Grenzflächen der dünnen Schicht reflektierten und/oder durch die Schicht transmittierten Lichtanteils wird ein farbsensitiver ortsauflösender Detektor verwendet, der die mittels Reflexion an der dünnen Schicht oder Transmission durch die dünne Schicht zum Detektor gelangenden Lichtanteile in mehreren mit den Spektralbereichen auf der Einstrahlungsseite mindestens teilweise überlappenden Detektionswellenlängenbereichen ortsaufgelöst detektiert und in separat weiter verarbeitbare elektrische Signale wandelt, die von den Intensitäten in den Detektionswellenlängenbereichen abhängig sind. Die Detektionswellenlängenbereiche korrespondieren mit "Detektionskanälen" bzw. "Farbkanälen" des Detektors. Die Auswertung der den Detektionswellenlängenbereichen zugehörigen elektrischen Signale umfasst eine Analyse von Intensitätsverhältnissen in den für die Auswertung vorgesehenen Detektionswellenlängenbereichen.

Es wird mittels des farbsensitiven ortsauflösenden Detektors eine ortsauflösende Detektion durchgeführt. Das Verfahren bietet also eine Auflösung im Ortsraum. Dies bedeutet unter anderem, dass das Verfahren eine Ortsinformation erfassen kann in der Weise, dass eine Aussage darüber getroffen werden kann, wo (d.h. an welchem Ort) des mithilfe des Detektors erfassten Bereichs ein Bindungsereignis stattgefunden hat. Damit ergibt sich einerseits die Möglichkeit, eine Vielzahl unterschiedlicher Bindungen räumlich getrennt auf einem Substrat zu betrachten. Andererseits ermöglicht es, eine einzelne Bindungsart auf Homogenität zu überprüfen

Der Erfindung liegt unter anderem eine eingehende Analyse der bekannten Verfahren und deren potenzieller Beschränkungen zugrunde. Wie eingangs erwähnt, gab es beispielsweise schon Verfahren, die ortsaufgelöst Änderungen der spektralen Intensitätsverhältnisse durch sequenzielle Abfrage mindestens zweier unterschiedlicher Spektralbereiche erfassten (vgl. z.B. DE 198 30 727 A1). Durch die sequenzielle Aufnahme besteht jedoch im Allgemeinen kein fester Zusammenhang zwischen den Messungen in den jeweiligen Farbkanälen, wodurch die Empfindlichkeit beeinträchtigt sein kann. Zusätzlich wird die erzielbare Zeitauflösung mindestens halbiert.

Herkömmliche faseroptische Verfahren, bei denen Änderungen der spektralen Intensitätsverhältnisse an einer Faserspitze simultan in mindestens zwei unterschiedlichen Spektralbereichen erfasst wurden (vgl. EP 1 685 367 B1), zielen primär auf eine Auflösung im Frequenzraum bzw. im Wellenlängenraum. Es werden keine Möglichkeiten beschrieben, bei vertretbarem apparativen Aufwand ortsaufgelöst zu messen. Auch Hochdurchsatzmessungen mit hunderten oder tausenden paralleler Messungen scheinen nicht oder nicht sinnvoll möglich.

Verfahren gemäß der beanspruchten Erfindung unterliegen diesen Beschränkungen nicht. Durch die simultane und ortsaufgelöste Verwendung von mindestens zwei Detektionswellenlängenbereichen kann die Messung simultan in mehreren Detektionswellenlängenbereichen und zeitlich parallel an einer Vielzahl von individuellen Messpunkten bzw. Messstellen durchgeführt werden. Weiterhin kann der Dynamikumfang der Messung im Vergleich zum Stand der Technik deutlich gesteigert werden. Bei manchen Varianten kann ein Dynamikumfang von 32 Bit (binary digits), entsprechend 2x10³² Intensitätsabstufungen, oder mehr erreicht werden. Dadurch werden sogar kleinste Intensitätsvariationen detektierbar, die mit monochromatischen oder sequenziell aufgenommenen polychromatischen Verfahren nicht oder nur mit sehr großem Aufwand erreicht werden können. Ein weiterer Vorteil der simultanen Erfassung von mindestens zwei Detektionswellenlängenbereichen liegt in der relativen Unempfindlichkeit gegenüber Schwankungen der Intensität des eingestrahlten Lichtes und gegenüber globalen Schwankungen der Detektorempfindlichkeit, da solche Schwankungen sich in gleicher Weise auf alle Detektionswellenlängenbereiche auswirken und somit bei der Verhältnisbildung im Rahmen der Auswertung nicht zum Tragen kommen. Das Verfahren und das entsprechende Messsystem sind daher gegenüber solchen äußeren Störungen besonders robust.

Insgesamt kann das beanspruchte Verfahren als ein bis zu vierdimensionales Verfahren verstanden werden, bei dem zwei Dimensionen den Ort in der Probenebene auflösen, die dritte Dimension die Intensität pro Farbkanal erfasst und die vierte Dimension der spektralen Unterteilung entspricht.

Bei einer Weiterbildung werden nur die an der dünnen Schicht reflektierten Lichtanteile erfasst und ausgewertet. Eine entsprechende Vorrichtung arbeitet also ausschließlich in Reflexion. Hierdurch lassen sich höhere Empfindlichkeiten erzielen als bei einer prinzipiell ebenfalls möglichen Erfassung und Auswertung von durch die dünne Schicht transmittierten Lichtanteilen. Es ist jedoch prinzipiell möglich, nur die durch die dünne Schicht transmittierten Lichtanteile erfassen und auszuwerten.

Es ist auch möglich, Interferenzerscheinungen sowohl in den transmittierten als auch in den reflektierten Lichtanteilen zu erfassen und auszuwerten. Man kann dann die gleiche Information in zwei voneinander unabhängigen Messungen erhalten. Signale, die nicht in beiden Messmodi (in Transmission und in Reflexion) auftreten, können ignoriert werden. Damit kann praktisch hintergrundfrei gemessen werden. Allerdings werden die Signalhübe in Reflexion und Transmission in der Regel stark unterschiedlich sein, was jedoch ggf. über einen festen Faktor oder auf andere Weise kompensiert werden kann. Eine entsprechende Vorrichtung kann mit zwei separaten Detektoren (einer für Messung in Reflexion, der andere für Messung in Transmission) ausgestattet sein. Hierdurch ist um den Preis höherer Kosten eine optimierte Signalerfassung möglich. Es ist auch möglich, mit nur einem Detektor zu arbeiten. Beispielsweise kann das Licht über Umlenkeinrichtungen (Spiegel, Lichtleiter) auf einen gemeinsamen Detektor gelenkt werden. Man nutzt dann vorzugsweise pro Messmodus nur einen Bruchteil der aktiven Detektorfläche, z.B. die Hälfte der aktiven Fläche.

Gemäß einer Weiterbildung wird die dünne Schicht mit Lichtanteilen aus mindestens drei separaten Spektralbereichen bestrahlt und der Detektor detektiert die reflektierten (oder transmittierten) Lichtanteile in mindestens drei Detektionswellenlängenbereichen. Hierdurch kann ein besserer Dynamikumfang (zum Beispiel 48 Bit, entsprechend 2x10⁴⁸ Intensitätsabstufungen, oder mehr) erreicht werden. Dadurch sind höhere Empfindlichkeiten erzielbar.

Als besonders zweckmäßig hat es sich herausgestellt, wenn der Detektor die empfangenen (z.B. die an der dünnen Schicht reflektierten) Lichtanteile in genau drei diskreten Detektionswellenlängenbereichen detektiert. Hierdurch ist ein guter Kompromiss zwischen erzielbarer Robustheit und Empfindlichkeit einerseits und apparativem Aufwand sowie Auswertungsaufwand andererseits erzielbar. Vorzugsweise entsprechen die Detektionswellenlängenbereiche einem Rotanteil, einem Grünanteil und einem Blauanteil der detektierten Lichtanteile. Wird auf diese Weise im RGB-Farbraum gearbeitet, so können aussagekräftige Messungen in ausreichend voneinander separierten Detektionswellenlängenbereichen vorgenommen werden. Zudem können für die apparative Umsetzung im Rahmen des Messsystems optische Komponenten genutzt werden, die für Verwendungen im RGB-Farbraum optimiert sind.

Es ist möglich, dass zur Erzeugung des auf die dünne Schicht einfallenden Lichts mehrere diskrete Lichtquellen genutzt werden, deren Emissionsspektren den gewünschten separaten Spektralbereichen entsprechen und deren Lichtemissionen über geeignete optische Komponenten in einen gemeinsamen Lichtstrahl zusammengeführt werden. Beispielsweise können lichtemittierende Dioden (LEDs) oder Laserdioden mit entsprechender spektraler Emissionscharakteristik (z.B. im roten, grünen und blauen Teil des sichtbaren Spektrums) genutzt werden.

Vorzugsweise wird als Lichtquelle eine Weißlichtquelle mit einem kontinuierlichen Spektrum im sichtbaren Wellenlängenbereich verwendet. Dieses Spektrum enthält diejenigen Spektralbereiche, die für die Messung genutzt werden sollen, sowie ggf. weitere, für die Messung nicht benötigte Spektralbereiche.

Die nicht benötigten Spektralbereiche können auf der Einstrahlungsseite, d.h. zwischen der Weißlichtquelle und der dünnen Schicht, oder nach Interaktion mit der dünnen Schicht, insbesondere also an der reflektierten Strahlung, ausgefiltert oder auf andere Weise selektiert werden.

Bei einer Ausführungsform wird die dünne Schicht mit einem kontinuierlichen Spektrum im sichtbaren Wellenlängenbereich bestrahlt und das von der dünnen Schicht reflektierte oder transmittierte Licht wird vor der Auswertung so gefiltert bzw. spektral selektiert, dass nur mit den Detektionswellenlängenbereichen mindestens teilweise überlappende Wellenlängenbereiche zur Auswertung gelangen.

Es ist auch möglich, dass die dünne Schicht (DS) mit einem diskontinuierlichen Spektrum mit Lücken zwischen genutzten Spektralbereichen im sichtbaren Wellenlängenbereich bestrahlt wird, wobei genutzte Spektralbereiche des diskontinuierlichen Spektrums ganz oder teilweise mit den Detektionswellenlängenbereichen überlappen.

Zur Umsetzung dieser Variante können mehrere diskrete Lichtquellen genutzt werden, z.B. LEDs oder Laserdioden. Es ist auch möglich, als Lichtquelle eine Weißlichtquelle mit einem kontinuierlichen Spektrum im sichtbaren Wellenlängenbereich zu verwenden und nicht benötigten Spektralbereiche auf der Einstrahlungsseite, d.h. zwischen der Weißlichtquelle und der dünnen Schicht, auszufiltern.

Vorzugsweise wird das Verfahren so umgesetzt, dass als Detektor eine Farbkamera mit einem zweidimensionalen Bildsensor verwendet wird. Bei dem Bildsensor kann es sich beispielsweise um einen sogenannten Bayer-Sensor oder um einen Foveon-X3-Direktbildsensor handeln. Als Bayer-Sensor bezeichnet man einen Photosensor, der ähnlich einem Schachbrett mit einem Farbfilter überzogen ist, welcher nach einer bestimmten Flächenzuweisung das eintreffende Licht in Grünanteil, Rotanteil und Blauanteil filtert. Die spektrale Filterung in die bevorzugten Detektionswellenlängenbereiche des RGB-Farbraums findet also hier direkt am Bildsensor statt. Auch andere bekannte farbsensitive Bildsensoren, beispielsweise nach dem Prinzip des Foveon-X3-Sensors, sind verwendbar. Derartige Sensoren nutzen anstelle mehrerer nebeneinanderliegender, für unterschiedliche Farben empfindliche Pixel an jedem Bildpunkt bzw. Pixel drei übereinanderliegende Sensorelemente, um mit jedem Bildpunkt Farbinformationen spektral getrennt nach Detektionswellenlängenbereich aufzuzeichnen. Mithilfe einer Farbkamera mit einem zweidimensionalen Bildsensor sind ortsauflösende Messungen in zwei Dimensionen möglich, so dass gleichzeitig an mehreren Probenpositionen in einer Fläche gemessen werden kann. Dies ist besonders vorteilhaft für Anwendungen, bei denen Probenpositionen in einer Matrix angeordnet sind, wie z.B. sogenannten Microarrays.

Zweidimensional ortsaufgelöste Messungen können auch als bildgebende Verfahren bezeichnet werden. Diese Verfahren haben den Vorteil, dass Interferenzbilder von einer definierten Fläche aufgezeichnet werden. Alle Interaktionsstellen, die sich in dieser Fläche befinden, können analysiert werden. Dabei es ist es weitgehend beliebig, in welcher Anordnung und Anzahl sich die Interaktionsstellen in dieser Fläche befinden. Dadurch erreicht man zum einen eine hohe Parallelisierung und zum anderen eine große Flexibilität, die mit diskreten Messpunkten, die bei nicht-bildgebenden Verfahren vorliegen, nicht in dieser Form gegeben ist.

Für einfachere ortsauflösende Messungen (in einer einzigen Dimension) könnte ggf. auch eine Zeilenkamera oder ein anderer eindimensional ausgedehnter Sensor verwendet werden.

Für die Analyse von Intensitätsverhältnissen in den Detektionswellenlängenbereichen im Rahmen der Auswertung gibt es vielfältige Möglichkeiten, die alternativ oder kumulativ genutzt werden können. Beispielsweise ist es möglich, dass die Analyse des Interferenzsignals durch Bildung eines Verhältniswerts von Intensitätswerten aus zwei ausgewählten Detektionswellenlängenbereichen erfolgt. Es ist auch möglich, einen ersten Verhältniswert von Intensitätswerten aus einem ersten Paar von zwei ausgewählten Detektionswellenlängenbereichen zu bilden, weiterhin einen zweiten Verhältniswert von Intensitätswerten aus einem von dem ersten Paar unterschiedlichen zweiten Paar von zwei Detektionswellenlängenbereichen zu bilden und darauf basierend einen Verhältniswert des ersten und des zweiten Verhältniswerts zu bilden. Hierzu werden drei oder mehr separate Detektionswellenlängenbereiche genutzt. Es ist auch möglich, ein Intensitätswerte-Triple zu berechnen, welches einem Punkt in einem Farbraum entspricht, der durch einen ersten, einen zweiten und einen dritten Detektionswellenlängenbereich aufgespannt wird. Die Intensitätsverhältnisse werden dann durch einen Punkt in einem dreidimensionalen Farbraum repräsentiert.

Die Erfindung betrifft auch ein Messsystem zur markierungsfreien Messung molekularer Interaktionen an einer dünnen Schicht. Das Messsystem ist so konfiguriert, dass damit das Verfahren durchgeführt werden kann. Als Lichtquelle kann z.B. eine Weißlichtquelle genutzt werden. Vorzugsweise weist der Detektor eine Farbkamera mit einem zweidimensionalen Bildsensor auf.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
- Fig. 1: zeigt schematisch ein Ausführungsbeispiel eines Messsystems zur markierungsfreien Messung molekularer Interaktionen an einer dünnen Schicht;
- Fig. 2: zeigt ein Diagramm, aus dem Verschiebungen von Interferenzspektren bei Bindung von Substanzen an eine Oberfläche einer dünnen Schicht sowie Änderungen der Intensitätsverhältnisse in drei Detektionswellenlängenbereichen ersichtlich sind;
- Fig. 3: zeigt ein Diagramm, aus dem eine Verstimmung zwischen Wellenlängenbereichen der Beleuchtung und Detektionswellenlängenbereichen sowie eine daraus resultierende effektive spektrale Bandbreite des detektierten Interferenzlichts erkennbar sind; und
- Fig. 4: zeigt schematisch die Darstellung eines Messwerts generiert aus drei Detektionswellenlängenbereichen, wobei der Messwert als Punkt in einem dreidimensionalen Farbraum beschreibbar ist.

### DETAILLIERTE BESCHREIBUNG DER AUSFÜHRUNGSBEISPIELE

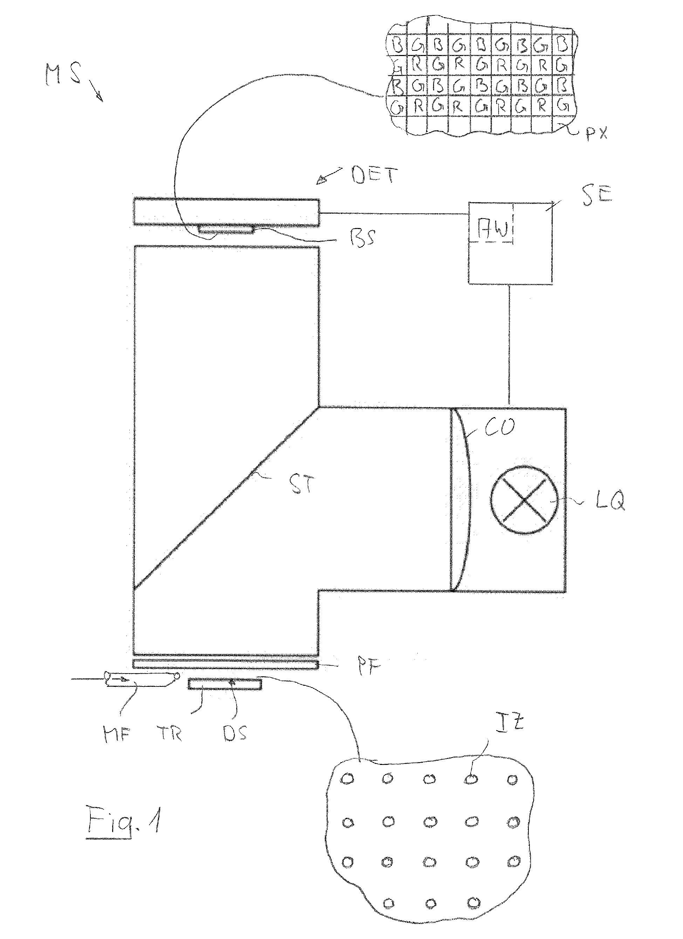

Die schematische Darstellung in Fig. 1 zeigt Komponenten eines Messsystems MS zur markierungsfreien Messung molekularer Interaktionen an einer dünnen Schicht DS, die auf einer ebenen Oberfläche eines Trägers TR angeordnet ist. Ein Bindungspartner (mindestens einer) der zu beobachteten Interaktion liegt in immobilisierter Form an der Oberfläche des Trägers vor und bildet dort eine dünne Schicht. Der andere Bindungspartner (mindestens einer) liegt in einem Trägerfluid verteilt vor. Das Trägerfluid mit den darin verteilten mobilen Bindungspartner(n) wird hier als "Probe" bezeichnet. Die Probe wird mittels geeigneter Mikrofluid-Einrichtungen MF in Kontakt mit dem an der Trägeroberfläche gebundenen (immobilisierten) Bindungspartner gebracht. Durch die Interaktion der immobilisierten Bindungspartner und der mit der Probe herangeführten Bindungspartner verändert sich die optische Schichtdicke der dünnen Schicht DS in charakteristischer Weise. Diese charakteristische Schichtdickenänderung führt bei Bestrahlung der Schicht mit Licht zu Veränderungen bei den Interferenzerscheinungen an der dünnen Schicht. Diese werden erfasst und ausgewertet.

Die immobilisierten Bindungspartner sind auf dem Träger TR in Form einer Vielzahl von kleinen, quasi punktförmigen Interaktionszonen IZ bzw. Interaktionsstellen aufgebracht, die z.B. in einer Rechteck-Matrixanordnung (siehe Detail) oder gemäß einem anderen regelmäßigen (z.B. Rautenmuster) oder unregelmäßigen Muster mit gegenseitigem Abstand zueinander angeordnet sind. Es kann sich um Hunderte oder Tausende oder Zehntausende von separaten Interaktionszonen handeln. Die Größe der Interaktionszonen kann stark variieren und beispielsweise im Bereich von ca. 10 µm Durchmesser bis zu einem oder mehreren Millimetern Durchmesser liegen. Limitierend ist hierbei nach oben vor allem die Größe des Messfensters des Detektors (typischerweise im Bereich von ein bis zwei Zentimetern Seitenlänge), nach unten die beugungsbegrenzte Abbildung bzw. die Pixelgröße der Kamera (z.B. einige Mikrometer). Es sind runde (kreisrund, oval etc.), polygonale oder irreguläre Formen der Interaktionszonen möglich. Die Interaktionszonen können hinsichtlich der immobilisierten Bindungspartner untereinander identisch oder unterschiedlich sein. Diese Anordnung wird hier auch als Microarray bezeichnet. Bei manchen Varianten können auf einer Fläche von einem Quadratzentimeter mehrere tausend solcher Interaktionszonen vorliegen. In jeder der Interaktionszonen liegt eine dünne Schicht der Interaktionspartner vor. Diese können zeitlich parallel bestrahlt und die Interferenzerscheinungen parallel ausgewertet werden.

Das Messsystem MS umfasst eine polychromatische Lichtquelle LQ zum Beleuchten der dünnen Schicht mit sichtbarem Licht, also elektromagnetischer Strahlung aus dem sichtbaren Wellenlängenbereich. Die polychromatische Lichtquelle gibt Licht mit einem kontinuierlichen Spektrum im sichtbaren Wellenlängenbereich ab. Die Lichtquelle kann beispielsweise eine Weißlicht-LED oder eine Halogenlampe aufweisen. Das Weißlicht wird über eine Kollimationsoptik CO nach Unendlich abgebildet und über einen Strahlteiler ST von oben auf die dünne Schicht DS gelenkt. Optional kann noch ein polarisationsdrehendes Phasenfilter PF vor der Probe P eingesetzt werden, um die Detektionseigenschaften für reflektiertes Licht zu verbessern.

Das Messsystem MS ist dafür ausgelegt, von der dünnen Schicht DS rückreflektiertes Interferenzlicht zu erfassen und auszuwerten. Das rückreflektierte Interferenzlicht durchtritt das Phasenfilter PF und den Strahlteiler ST und trifft auf die aktive Fläche eines Detektors DET, bei dem es sich um einen farbsensitiven ortssauflösenden Detektor in Form einer RGB-Farbkamera handelt. Der Detektor hat einen zweidimensionalen Bildsensor BS mit einer Pixelanordnung, bei der gemäß dem Bayer-Schema ein Teil der Pixel PX im blauen Wellenlängenbereich (B), ein Teil der Pixel im grünen Wellenlängenbereich (G) und ein Teil der Pixel im roten Wellenlängenbereich (R) sensitiv ist (siehe Detail). Die Pixel sind den drei Detektionskanälen bzw. Farbkanälen des Detektors zugeordnet. Ein erster Detektionskanal D1 korrespondiert mit einem ersten Detektionswellenlängenbereich mit Wellenlängen im blauen Teils des Spektrums, ein zweiter Detektionskanal D2 korrespondiert mit einem spektral versetzt liegenden zweiten Detektionswellenlängenbereich mit Wellenlängen im grünen Teils des Spektrums und ein dritter Detektionskanal D3 korrespondiert mit einem spektral versetzt liegenden dritten Detektionswellenlängenbereich mit Wellenlängen im roten Teils des Spektrums.

Die Ebene, in der die dünne Schicht bzw. die Matrix mit den Interaktionszonen angeordnet ist, wird mittels eines optischen Abbildungssystems optisch auf die Ebene der aktiven Fläche des Detektors DET abgebildet in der Weise, dass Bilder der räumlich separaten Interaktionszonen auf räumlich separate Bereiche der aktiven Fläche fallen und somit durch unterschiedliche Pixelgruppen erfasst werden. Dadurch ist eine ortsauflösende Detektion der Ereignisse an der dünnen Schicht möglich, wobei die Ereignisse an jeder der Interaktionszonen unabhängig von den Ereignissen anderer Interaktionszonen, jedoch zeitgleich mit diesen, erfasst und spektral sowie hinsichtlich ihrer Intensität ausgewertet werden können.

Der Detektor bzw. die Kamera ist an die Steuereinheit SE des Messsystems MS angeschlossen, die auch die Lichtquelle LQ steuert. Die Steuereinheit umfasst eine Auswerteeinrichtung AW zum Auswerten der elektrischen Signale des Detektors DET, um daraus Parameter zu ermitteln, die die zu untersuchenden Interaktionen an der dünnen Schicht DS charakterisieren. Die Auswertung umfasst eine Analyse von Intensitätsverhältnissen in den Detektionswellenlängenbereichen des Detektors DET.

Mithilfe des Messsystems MS lässt sich ein kamerabasiertes Verfahren zur Weißlichtinterferenzspektroskopie durchführen. Bei dem Verfahren wird die dünne Schicht, die in einem Mikroarray-Format vorliegen kann, mit Weißlicht der Lichtquelle LQ beaufschlagt. Das vom Schichtsystem rückreflektierte Licht wird ortsauflösend mit dem Detektor DET erfasst, der hier in Form einer farbsensitiven Kamera vorliegt, die die auf die aktive Fläche auftreffende Strahlung in drei voneinander separaten Detektionswellenlängenbereichen, nämlich rot (R), grün (G) und blau (B) erfasst. Die drei Detektionswellenlängenbereiche entsprechen drei Detektionskanälen, in denen separate, von den Intensitäten in den drei Detektionswellenlängenbereichen abhängige elektrische Signale vorliegen, die zur Auswerteeinrichtung AW übermittelt und dort ausgewertet werden.

Eine gegebene Weißlichtinterferenz an der dünnen Schicht DS erzeugt ein definiertes Intensitätsverhältnis in den drei Farbkanälen bzw. Detektionskanälen. Eine durch Änderungen der optischen Schichtdicke der dünnen Schicht verursachte Änderung der Interferenz am Probenschichtsystem führt zu einer spektralen Verschiebung des Interferenzspektrums und dadurch zu einer Veränderung der relativen Intensitätsverhältnisse der drei Farbkanäle bzw. Detektionskanäle. Die Veränderung der Intensitätsverhältnisse ist proportional zur spektralen Verschiebung des Interferenzspektrums und damit zur Änderung der optischen Schichtdicke der dünnen Schicht. Durch Analyse der Intensitätsverhältnisse in den drei Farbkanälen bzw. Detektionskanäle kann somit eine durch molekulare Interaktionen hervorgerufene Schichtdickenänderung verfolgt und quantitativ erfasst und ausgewertet werden.

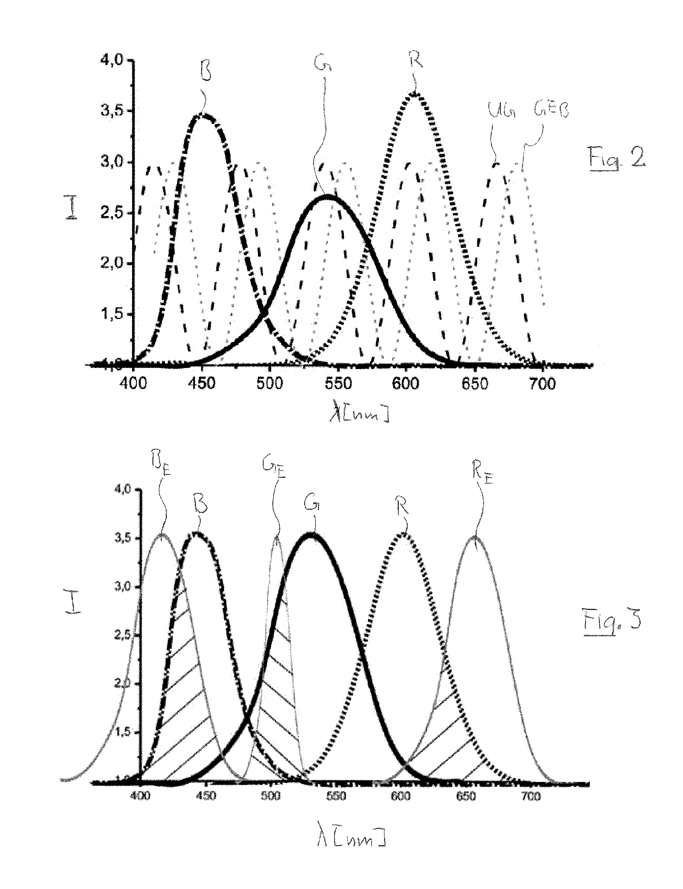

Die RGB-Farbkamera des Detektors DET weist eine Farbraumkodierung auf, die sich durch normierte Empfindlichkeitskurven für die drei Farbkanäle rot (R), grün (G) und blau (B) darstellen lässt, beispielsweise durch eine Farbraumcodierung nach CIE 1931. Fig. 2 zeigt beispielhaft drei CIE-genormte Empfindlichkeitskurven B (mit einer Zentralwellenlänge bei ca. 430 nm), G (mit einer Zentralwellenlänge von ca. 530 nm) und R (mit einer Zentralwellenlänge bei ca. 605 nm).

Überlagert man diese spektralen Empfindlichkeitskurven mit typischen Interferenzspektren (Abhängigkeit der Intensität I von der Wellenlänge λ) für unterschiedliche Schichtdicken der dünnen Schicht DS, so ist aus Fig. 2 gut zu erkennen, dass sich die integrierte Intensität pro Farbkanal in Abhängigkeit vom Interferenzspektrum bzw. von den spektralen Positionen der Interferenzmaxima und Interferenzminima ändert. In Fig. 2 repräsentiert die gestrichelte Linie UG die Lage des Interferenzspektrums im ungebundenen Zustand und die Kurve GEB das demgegenüber spektral verschobene Interferenzspektrum bei Bindung von Substanzen an einer Oberfläche.

Jedem der Detektionskanäle bzw. der Detektionswellenlängenbereiche kann für jede Lage eines Interferenzspektrums ein bestimmter Intensitätswert zugeordnet werden, der der Intensität des Spektrums im jeweiligen Detektionskanal bzw. Farbkanal entspricht. Die Intensitätswerte der Farbkanäle können paarweise und/oder in Gruppen zueinander ins Verhältnis gesetzt werden, zum Beispiel in Form einer Intensitätsmatrix. Auf diese Weise ist für jede Ausprägung des Interferenzspektrums bzw. für jede spektrale Lage eine individuelle Zahlenkombination ermittelbar, die spezifisch für die Intensitätsverteilung in den einzelnen Detektionskanälen und damit für die spektrale Lage des Interferenzspektrums ist.

Die Dynamik der Auslesung und damit die Empfindlichkeit der Messung hängen unter anderem von der Intensitätsquantisierung der Daten ab. Bei Auswertung von drei Detektionskanälen kann beispielsweise eine Quantisierung von 48 Bit (je 16 Bit für jeden der drei Detektionskanäle) erreicht werden. Dadurch kann eine farbselektive kamerabasierte Weißlichtinterferenzdetektion der hier vorgeschlagenen Art einer entsprechenden monochromatischen Detektion hinsichtlich Empfindlichkeit deutlich überlegen sein, da auch sehr kleine Schichtdickenänderungen zuverlässig erfasst und von Hintergrundsignalen separiert werden können. Eine derartige Messwerterfassung und Auswertung kann aufgrund der ortsauflösenden Detektion für eine Vielzahl nebeneinander vorliegender Interaktionszonen gleichzeitig (simultan) erfolgen.

Fig. 3 zeigt eine Belichtung mit drei voneinander separierten Wellenlängenbereichen, die nur partiell mit den Detektionsfenstern (Detektionswellenlängenbereichen) der Kamera überlappen. Damit wird der effektive Spektralbereich pro Farbkanal (schraffierter Überlappungsbereich). Kleiner. Das führt zu einer größeren Kohärenzlänge des Lichts und ermöglicht damit größere Eindringtiefen, was z.B. für die Messung von zellulären Systemen wichtig ist.

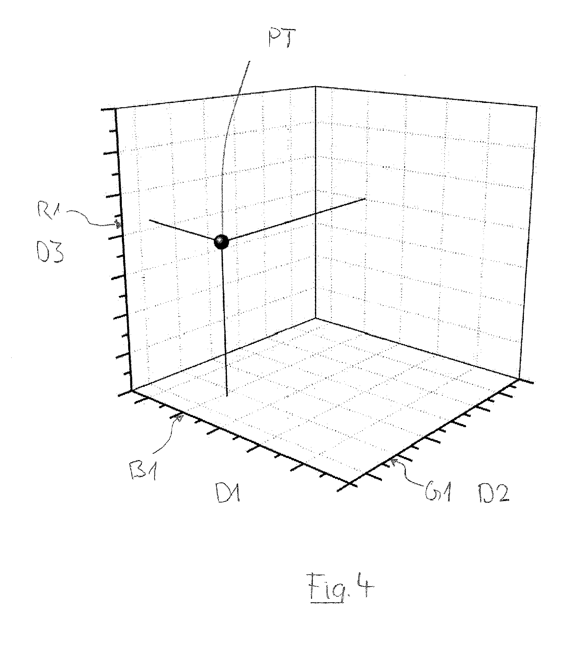

Anhand von Fig. 4 wird eine von zahlreichen Möglichkeiten zur Analyse von Intensitätsverhältnissen in den Detektionswellenlängenbereichen dargestellt. Das dreidimensionale Diagramm in Fig. 4 stellt einen RGB-Farbraum dar, dessen zueinander orthogonale Achsen zu den drei Detektionswellenlängenbereichen bzw. Detektionskanälen D1 für blau (B), D2 für grün (G) und D3 für rot (R) gehören. Im blauen Farbkanal liege die Intensität B1 vor, im grünen Farbkanal die Intensität G1 und im roten Farbkanal die Intensität R1. Diese für die optische Schichtdicke des Schichtsystems spezifische Intensitätswertkombination wird im Farbraum durch den Punkt PT repräsentiert. Es ist unmittelbar ersichtlich, dass auch sehr kleine Änderungen in den Intensitätsverhältnissen der Detektionskanäle untereinander zu einem anderen Intensitätswert-Triple bzw. zu einer signifikant anderen Lage des resultierenden Punkts im (dreidimensionalen) Farbraum führen würden. Dies veranschaulicht die hohe Empfindlichkeit des Verfahrens auch bei sehr kleinen Änderungen der Schichtdicke.

Bei Verwendung einer Weißlichtquelle, deren kontinuierliches Spektrum alle drei Detektionswellenlängenbereiche enthält, ist automatisch eine optimale Nutzung der Empfindlichkeiten in den Detektionskanälen gegeben, da Einstrahlungswellenlängenbereiche genutzt werden, die mit den Detektionswellenlängenbereichen übereinstimmen.

Es kann jedoch auch Situationen geben, in denen diese Übereinstimmung nur partiell gegeben ist. Dies kann beispielsweise bei Ausführungsformen sein, bei denen diskrete Strahlungsquellen für den blauen, grünen und roten Bereich verwendet werden, deren Emissionsspektren R_{E}, G_{E} und R_{E} nicht vollständig mit den Empfindlichkeitsspektren R, G und B auf der Detektorseite übereinstimmen (vgl. Fig. 3). Ist somit die Übereinstimmung zwischen Einstrahlungswellenlängenbereich und Detektionswellenlängenbereich nur partiell gegeben (in den schraffiert dargestellten Bereichen der Überlappung zwischen Empfindlichkeitskurven und Emissionsspektren), so lässt sich dennoch eine effektive spektrale Breite des pro Detektionswellenlängenbereich erfassten Lichts eingrenzen und damit dessen Kohärenzlänge verändern. Die Kohärenzlänge hängt unter anderem von der spektralen Breite des Lichts ab. Je schmaler diese ist, bei desto mehr Wellenzügen besteht ein definiertes Phasenverhältnis zwischen zwei Teilstrahlen, wodurch die Interferenzfähigkeit gegeben ist. Dadurch kann die maximale Schichtdicke, bei der Änderungen interferometrisch erfasst werden können, präzise eingestellt und der gewünschten Anwendung angepasst werden. Diese Anpassung kann beispielsweise vorteilhaft sein, wenn zelluläre Proben vermessen werden, bei denen Schichtdickenänderungen von bis zu mehreren zehn Mikrometern auftreten können.

Bei manchen Ausführungsformen sind die diskreten Einstrahlungswellenlängen und/oder deren spektrale Bandbreite nicht fest vorgegeben, sondern lassen sich in einem bestimmten Bereich einstellen, so dass der Arbeitsbereich durch den Nutzer kontinuierlich einstellbar ist. Das ist im Prinzip der gleiche Effekt: Beispielsweise kann einen dreifach Bandpassfilter hinter eine Weißlichtquelle gestellt bzw. angeordnet werden. Durch die Wahl des Filters lässt der Grad der Überlappung zwischen Anregung und Detektion einstellen. Damit kann man dann die Kohärenzlänge und damit die Eindringtiefe einstellen. Hierbei können die Kohärenzlängen für die einzelnen Detektionswellenlängen unterschiedlich eingestellt werden, so dass sich für diese Detektionskanäle unterschiedliche Eindringtiefen ergeben. Eine solche Anordnung ist besonders dann vorteilhaft, wenn bei einem gegebenen Probensystem parallel verschiedene Bindungsereignisse, die bei unterschiedlichen Eindringtiefen stattfinden, beobachtet werden sollen. Dies ist z.B. bei der Vermessung zellulärer Systeme vorteilhaft, bei denen das direkte Bindungssignal an der Zelloberfläche von nachgeschalteten Effekten, die im Zellinneren zu einer Umverteilung innerzellulärer Kompartimente führen, differenziert werden soll.

## Patentansprüche

1. Verfahren zur markierungsfreien Messung molekularer Interaktionen an einer dünnen Schicht (DS) mit folgenden Schritten:
Beleuchten der dünnen Schicht (DS) mit Licht einer Lichtquelle (LQ);
Erfassen eines an Grenzflächen der dünnen Schicht reflektierten oder transmittierten Lichtanteils mittels eines Detektors (DET), welcher den erfassten Lichtanteil in elektrische Signale wandelt;
Auswerten der elektrischen Signale zur Ermittlung von Parametern, die die zu untersuchenden Interaktionen charakterisieren;
**dadurch gekennzeichnet, dass**
die dünne Schicht (DS) mit polychromatischem Licht bestrahlt wird, das gleichzeitig Lichtanteile aus mehreren separaten Spektralbereichen enthält;
ein farbsensitiver ortsauflösender Detektor (DET) verwendet wird, der die an der dünnen Schicht (DS) reflektierten und/oder durch die dünne Schicht transmittierten Lichtanteile in mehreren mit den Spektralbereichen mindestens teilweise überlappenden Detektionswellenlängenbereichen (D1, D2, D3) ortsaufgelöst detektiert und in separate, von den Intensitäten in den Detektionswellenlängenbereichen abhängige elektrische Signale wandelt; und
die Auswertung der Signale eine Analyse von Intensitätsverhältnissen in den Detektionswellenlängenbereichen umfasst.

2. Verfahren nach Anspruch 1, worin nur die an der dünnen Schicht (DS) reflektierten Lichtanteile erfasst und ausgewertet werden.

3. Verfahren nach Anspruch 1 oder 2, worin die dünne Schicht (DS) mit Lichtanteilen aus mindestens drei separaten Spektralbereichen bestrahlt wird und der Detektor die empfangenen Lichtanteile in mindestens drei Detektionswellenlängenbereichen (D1, D2, D3) detektiert.

4. Verfahren nach einem der vorhergehenden Ansprüche, worin der Detektor (DET) die empfangenen Lichtanteile in genau drei diskreten Detektionswellenlängenbereichen (D1, D2, D3) detektiert, wobei die Detektionswellenlängenbereiche vorzugsweise einen Rotanteil (R), einen Grünanteil (G) und einen Blauanteil (B) der detektierten Lichtanteile repräsentieren.

5. Verfahren nach einem der vorhergehenden Ansprüche, worin als Lichtquelle (LQ) eine Weißlichtquelle mit einem kontinuierlichen Spektrum im sichtbaren Wellenlängenbereich verwendet wird.

6. Verfahren nach einem der vorhergehenden Ansprüche, worin die dünne Schicht (DS) mit einem kontinuierlichen Spektrum im sichtbaren Wellenlängenbereich bestrahlt wird und worin die von der dünnen Schicht reflektierte und/oder transmittierte Strahlung vor der Auswertung in mit den Detektionswellenlängenbereichen mindestens teilweise überlappende Wellenlängenbereiche gefiltert wird.

7. Verfahren nach einem der Ansprüche 1 bis 5, worin die dünne Schicht (DS) mit einem diskontinuierlichen Spektrum mit Lücken zwischen genutzten Spektralbereichen im sichtbaren Wellenlängenbereich bestrahlt wird, wobei genutzte Spektralbereiche des diskontinuierlichen Spektrums ganz oder teilweise mit den Detektionswellenlängenbereichen überlappen.

8. Verfahren nach der Anspruch 7, worin als Lichtquelle eine Weißlichtquelle mit einem kontinuierlichen Spektrum im sichtbaren Wellenlängenbereich verwendet wird und nicht benötigte Spektralbereiche zwischen der Weißlichtquelle und der dünnen Schicht ausgefiltert werden.

9. Verfahren nach einem der vorhergehenden Ansprüche, worin als Detektor (DET) eine Farbkamera mit einem zweidimensionalen Bildsensor (BS) verwendet wird.

10. Verfahren nach einem der vorhergehenden Ansprüche, worin eine parallelisierte Messung durchgeführt wird, bei der gleichzeitig eine Vielzahl von Interaktionszonen (IZ) bestrahlt und Information aus den Interaktionszonen zweidimensional ortsaufgelöst erfasst und in den mehreren Detektionswellenlängenbereichen ausgewertet wird.

11. Verfahren nach einem der vorhergehenden Ansprüche, worin die Auswertung mindestens einen der folgenden Schritte umfasst:
(i) Bildung eines Verhältniswerts von Intensitätswerten aus zwei Detektionswellenlängenbereichen;
(ii) Bildung eines ersten Verhältniswerts von Intensitätswerten aus einem ersten Paar von zwei Detektionswellenlängenbereichen, Bildung eines zweiten Verhältniswerts von Intensitätswerten aus einem von dem ersten Paar unterschiedlichen zweiten Paar von zwei Detektionswellenlängenbereichen und Bildung eines Verhältniswertes des ersten und des zweiten Verhältniswertes;
(iii) Berechnen eines Intensitätswertetripels, welches einen Punkt in einem Farbraum entspricht, der durch einen ersten, einen zweiten und einen dritten Detektionswellenlängenbereich aufgespannt wird.

12. Messsystem zur markierungsfreien Messung molekularer Interaktionen an einer dünnen Schicht (DS) umfassend:
eine Lichtquelle (LQ) zum Beleuchten der dünnen Schicht (DS) mit Licht;
einen Detektor (DET) zum Erfassen eines an Grenzflächen der dünnen Schicht reflektierten oder durch die dünne Schicht transmittierten Lichtanteils und zum Wandeln des erfassten Lichtanteils in elektrische Signale;
einer Auswerteeinrichtung zum Auswerten der elektrischen Signale zur Ermittlung von Parametern, die die zu untersuchenden Interaktionen charakterisieren;
**dadurch gekennzeichnet, dass**
die Lichtquelle (LQ) für die Erzeugung polychromatischen Lichts ausgelegt ist, das gleichzeitig Lichtanteile aus mehreren separaten Spektralbereichen enthält;
der Detektor ein farbsensitiver ortsauflösender Detektor (DET) ist, durch den die an der dünnen Schicht reflektierten oder durch die dünne Schicht transmittierte Lichtanteile in mehreren mit den Spektralbereichen mindestens teilweise überlappenden Detektionswellenlängenbereichen (D1, D2, D3) ortsaufgelöst detektierbar und in separate, von den Intensitäten in den Detektionswellenlängenbereichen abhängige elektrische Signale wandelbar sind; und
die Auswerteeinrichtung (AW) für eine Auswertung der Signale konfiguriert ist, wobei die Auswertung eine Analyse von Intensitätsverhältnissen in den Detektionswellenlängenbereichen umfasst.

13. Messsystem nach Anspruch 12, worin der Detektor (DET) eine Farbkamera mit einem zweidimensionalen Bildsensor (BS) aufweist.

14. Messsystem nach Anspruch 12 oder 13, worin die Auswerteeinrichtung dafür konfiguriert ist, mindestens eine der folgenden Auswertungen durchzuführen:
(i) Bildung eines Verhältniswerts von Intensitätswerten aus zwei Detektionswellenlängenbereichen;
(ii) Bildung eines ersten Verhältniswerts von Intensitätswerten aus einem ersten Paar von zwei Detektionswellenlängenbereichen, Bildung eines zweiten Verhältniswerts von Intensitätswerten aus einem von dem ersten Paar unterschiedlichen zweiten Paar von zwei Detektionswellenlängenbereichen und Bildung eines Verhältniswertes des ersten und des zweiten Verhältniswertes;
(iii) Berechnen eines Intensitätswertetripels, welches einen Punkt in einem Farbraum entspricht, der durch einen ersten, einen zweiten und einen dritten Detektionswellenlängenbereich aufgespannt wird.
